# EUROPEAN PATENT SPECIFICATION

(11) **EP 2 711 040 B1**
(45) Date of publication and mention of the grant of the patent: **27.04.2016**
(21) Application number: 12785308.3
(22) Date of filing: 14.05.2012
(51) Int. Cl.: A61M 16/04, A61F 5/56, A61M 16/06, A61F 5/08

(54) **CLIP FOR NASAL INTUBATION DEVICE**
KLAMMER FÜR EINE NASALE INTUBATIONSVORRICHTUNG
ATTACHE POUR DISPOSITIF D'INSERTION DANS UNE CAVITÉ NASALE

(30) Priority: 13.05.2011 JP 2011108839
(43) Date of publication of application: 26.03.2014
(73) Proprietor: seven dreamers laboratories, Inc., Tokyo (JP)
(72) Inventor: HIOKI, Kenji, Otsu-shi Shiga 520-2153 (JP); YAMADA, Hiroshi, Otsu-shi Shiga 520-2153 (JP)
(74) Representative: Horn Kleimann Waitzhofer Patentanwälte PartG mbB
(86) International application number: PCT/JP2012/003128
(87) International publication number: WO 2012/157243

(56) References cited:
- EP-A1- 2 543 407
- WO-A1-00/09192
- WO-A1-2006/047811
- WO-A1-2010/113305
- WO-A1-2011/108253
- WO-A1-2011/108282
- JP-A- 2001 231 860
- JP-A- 2009 072 582
- JP-A- 2009 291 615
- JP-A- 2010 154 918
- US-A- 3 915 173
- US-A- 5 105 807
- US-A- 5 895 409
- US-A- 5 931 852
- US-A1- 2010 042 134

## Description

### TECHNICAL FIELD

The present invention relates to a nasal cavity insertion device fixture.

### BACKGROUND ART

Patients of obstructive sleep apnea syndrome (OSAS) intermittently repeat a temporally suffocated state (apnea, infrequent breathing) while the pharynx of the upper respiratory tract is obstructed due to a complication of muscle relaxation and obesity and the like during sleep. Therefore, patients of OSAS are suffered from hypertension or disorders in brain blood vessels and cardiac blood vessels. Further, patients of OSAS cannot sleep sufficiently, and therefore, tend to feel drowsy in the daytime and/or tend to lack of concentration or vitality in the daytime. Yet further, when driving a car, patients of OSAS have high chances of causing an accident, a serious accident or the like due to falling asleep at the wheel.

On the other hand, similarly to OSAS, snoring occurs when the respiratory tract's mucus membranes of the pharynx and the like vibrate due to stenosis or obstruction of the upper respiratory tract during sleep. Snoring disturbs not only the sleep of a roommate but also the sound sleep of a snorer oneself, and the snorer tends to feel drowsy in the daytime or tends to lack of concentration or vitality in the daytime.

The aforementioned "obstructive sleep apnea syndrome" and "snoring syndrome" will be hereinafter collectively referred to as "sleep disorder".

In view of the above, a variety of proposals have been made in recent years for treating or resolving OSAS. For example, such proposals include "a method of inserting an obstructive sleep apnea syndrome resolving tube into the pharynx via a nasal passage (see e.g., Japan Laid-open Patent Application Publication No. JP-A-2009-072581) ", "a method of expanding the pharynx by inserting an obstructive sleep apnea syndrome resolving tube, to the tip of which a water swelling resin is applied, into the pharynx via a nasal passage, and subsequently, by swelling the water swelling resin using moisture in the periphery of the pharynx (see e.g., Japan Laid-open Patent Application Publication No. JP-A-2009-072581) ", "a method of expanding the pharynx by inserting an obstructive sleep apnea syndrome resolving tube having an expansion portion in the tip thereof into the pharynx via a nasal passage, and subsequently, by expanding the expansion portion through a user's operation (see e.g., Japan Laid-open Patent Application Publications Nos. JP-A-2006-204630, JP-A-2009-034384, JP-A-2009-072581, JP-A-2009-072582, etc.)" and etc.

### CITATION LIST

### PATENT LITERATURE

PTL1: Japan Laid-open Patent Application Publication No. JP-A-2006-204630
PTL2: Japan Laid-open Patent Application Publication No. JP-A-2009-034384
PTL3: Japan Laid-open Patent Application Publication No. JP-A-2009-072581
PTL4: Japan Laid-open Patent Application Publication No. JP-A-2009-072582

### SUMMARY OF INVENTION

The invention is defined in claim 1. A preferred embodiment is defined in claim 2. The closet prior art, forming the basis for the two-part form, is disclosed in US-A-5 105 807.

### TECHNICAL PROBLEM

Incidentally, in the aforementioned methods, it has been concerned that a patient of sleep disorder is not treated sufficiently due to the displacement or detachment of the nasal cavity insertion tube during the sleep of the patient of sleep disorder.

It is an object of the present invention to provide a nasal cavity insertion device fixture and a nasal cavity insertion device set including the nasal cavity insertion device fixture, whereby the displacement or detachment of a nasal cavity insertion tube can be prevented during the sleep of a patient of sleep disorder.

### SOLUTION TO PROBLEM

A nasal cavity insertion device fixture according to a first aspect of the present invention fixes a device to be inserted into a nasal cavity by means of a force of expanding a nasal passage from inside the nasal passage.

According to the aforementioned configuration, the device can be fixed by means of the force of expanding a nasal passage from the inside of the nasal passage. Therefore, the displacement or detachment of the device can be prevented during the sleep of a patient of sleep disorder.

Further, the nasal cavity insertion device fixture is disposed inner the nasal passage. Therefore, the nasal cavity insertion device fixture can be prevented from standing out.

Yet further, the attached position of the nasal cavity insertion device fixture is the nose. Therefore, a user can attach the nasal cavity insertion device fixture thereto by oneself.

A nasal cavity insertion device fixture according to a second aspect of the present invention relates to the nasal cavity insertion device fixture according to the first aspect, and at least makes contact with an inner surface of a nasal wing.

According to the aforementioned configuration, the device can be fixed by means of a force to make contact with the nasal wing.

A nasal cavity insertion device fixture according to a third aspect of the present invention relates to the nasal cavity insertion device fixture according to the first or second aspect, and includes a tubular contact portion that an outer peripheral surface thereof makes contact with an inner surface of the nasal passage.

According to the aforementioned configuration, the outer peripheral surface of the tubular contact portion makes contact with the inner surface of the nasal passage. The device can be thereby firmly fixed. Therefore, the displacement or detachment of the device can be prevented during the sleep of a patient of sleep disorder.

Further, the contact portion is formed in a tubular shape that allows the air to path therethrough. Therefore, hindrance to the respiration of a patient by the nasal cavity insertion device fixture can be suppressed.

A nasal cavity insertion device fixture according to a fourth aspect of the present invention relates to the nasal cavity insertion device fixture according to the third aspect, and wherein the tubular contact portion is capable of accommodating therein the device.

According to the aforementioned configuration, the device can be accommodated inside the tubular contact portion. Therefore, a single nasal passage can be commonly used as the nasal passage into which the device is inserted and the nasal passage into which the nasal cavity insertion device fixture is inserted. A normal nasal cavity, free from occurrence of obstruction of the pharynx, can be thereby kept open. Therefore, the nasal cavity insertion device fixture can be prevented from hindering the respiration of a patient.

A nasal cavity insertion device fixture according to a fifth aspect of the present invention relates to the nasal cavity insertion device fixture according to the first or second aspect, and includes: a first contact portion making contact with an inner surface of one nasal wing; a second contact portion making contact with an inner surface of the other nasal wing; and a coupling portion coupling the first contact portion and the second contact portion so as to cause the first contact portion to make contact with the inner surface of the one nasal wing with a predetermined contact force and cause the second contact portion to make contact with the inner surface of the other nasal wing with a predetermined contact force.

According to the aforementioned configuration, the nasal cavity insertion device fixture is fixed to the both nasal passages. Therefore, the nasal cavity insertion device fixture and the device mounted to the nasal cavity insertion device fixture can be firmly fixed.

A nasal cavity insertion device fixture according to a sixth aspect of the present invention relates to the nasal cavity insertion device fixture according to the fifth aspect, and wherein the first contact portion, the second contact portion and the coupling portion are formed by a single flat spring.

According to the aforementioned configuration, the nasal cavity insertion device fixture, including the first contact portion, the second contact portion and the coupling portion, can be formed by the single flat spring. In other words, the nasal cavity insertion device fixture can be simply structured. Accordingly, in reusing the nasal cavity insertion device fixture, the nasal cavity insertion device fixture can be easily cleaned and can be used in a sanitary manner.

A nasal cavity insertion device fixture according to a seventh aspect of the present invention relates to the nasal cavity insertion device fixture according to the first or second aspect, and includes a swell portion that an outer peripheral surface thereof makes contact with an inner surface of the nasal passage, and wherein the swell portion allows an air to pass therethrough.

According to the aforementioned configuration, the swell portion makes contact with the inner wall of the nasal passage. Therefore, the device can be firmly fixed. In this case, the swell portion allows the air to pass therethrough. Therefore, even when the swell portion is inserted into the nasal passage, hindrance to the respiration of a patient by the nasal cavity insertion device fixture can be suppressed.

A nasal cavity insertion device fixture according to an eighth aspect of the present invention relates to the nasal cavity insertion device fixture according to the seventh aspect. The swell portion is a reticular member.

According to the aforementioned configuration, the swell portion is designed as a reticular member. Accordingly, the air is allowed to pass therethrough with a simple structure.

A nasal cavity insertion device set according to a ninth aspect of the present invention includes the nasal cavity insertion device fixture according to any of the aforementioned aspects and the device to be inserted into the nasal cavity.

According to the aforementioned configuration, the device can be fixed with a contact force applied to the inner surface of the nasal passage. Therefore, the displacement or detachment of the device can be prevented during the sleep of a patient of sleep disorder.

### BRIEF DESCRIPTION OF THE DRAWINGS

FIG. 1 is a plan view of an entire structure of a nasal cavity insertion device set.
FIG. 2 is a schematic diagram showing a state that the nasal cavity insertion device set shown in FIG. 1 is inserted into a nasal passage.
FIG. 3 is a schematic diagram showing a state that a nasal cavity insertion device set inserted into a nasal passage.
FIG. 4 is a plan view of a nasal cavity insertion device set in accordance with the present invention.
FIG. 5 is a perspective view of a nasal cavity insertion device set according to a reference example.

### REFERENCE SIGNS LIST

1, 1a, 1b Nasal cavity insertion device set
100 Fixture (tubular contact portion)
100a Fixture
110a First curved portion (first contact portion)
120a Second curved portion (second contact portion)
130a Coupling portion
100b Fixture (swell portion)
200 Nasal cavity insertion tube

### DESCRIPTION OF EMBODIMENTS

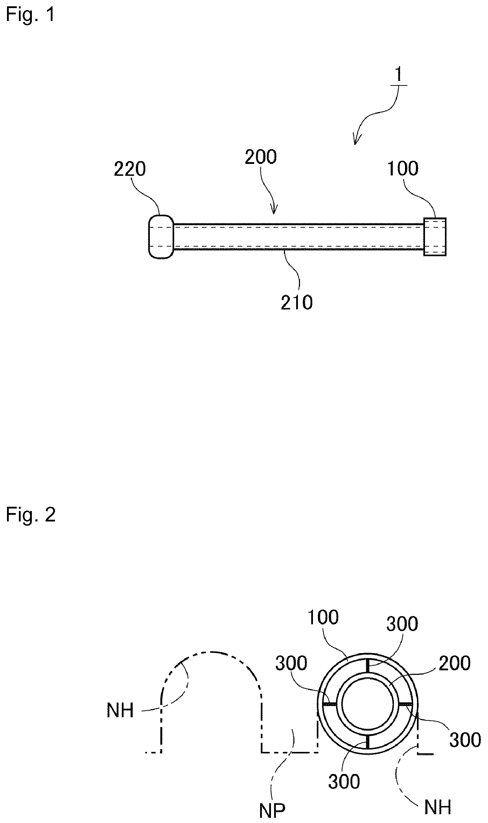

As shown in FIG. 1, a nasal cavity insertion device set 1 according to an embodiment of the present invention mainly includes a nasal cavity insertion tube 200 and a nasal cavity insertion device fixture (hereinafter simply referred to as "a fixture" on an as-needed basis) 100 for fixing the nasal cavity insertion tube 200 to the nose.

### <Nasal Cavity Insertion Tube>

The nasal cavity insertion tube 200 takes the form of a tubular body made of silicone resin or thermoplastic elastomer (of styrene series, vinyl chloride series, olefin series, urethane series, polyester-series, etc.), and is formed such that it can be extended from a nasal passage to the pharynx. The nasal cavity insertion tube 200 has: a main body portion 210 formed in a round tubular shape; and an expansion portion 220 mounted to the tip end (pharynx side) of the main body portion 210. The main body portion 210 serves a function of forming a flow path of the air when the nasal cavity insertion tube 200 is attached to the nasal cavity. On the other hand, the expansion portion 220 is provided for expanding the pharynx, and expands the potentially narrowed/obstructed pharynx of a patient of sleep disorder. Further, in the present embodiment, the fixture 100 is mounted to the base end (nasal passage side) of the nasal cavity insertion tube 200.

### <Fixture>

As shown in FIG. 2, the fixture 100 according to the present embodiment makes contact with the inner surface of a nasal passage NH in order to fix the nasal cavity insertion tube 200 connected to the fixture 100. The fixture 100 is a deflectable member and takes the form of a tubular body made of silicone resin. The outer diameter of the fixture 100 is greater than the inner diameter of the nasal passage NH so that the outer peripheral surface of the fixture 100 can make contact with the inner surface of the nasal passage NH. The size of the aforementioned inner diameter of the nasal passage NH depends on patients. Therefore, it is preferable to select the outer diameter of the fixture suitable for a patient on an as-needed basis. When inserted into the nasal passage NH, the fixture 100 is inserted into the nasal passage NH while the diameter thereof is reduced by compression. In turn, the fixture 100 inserted into the nasal passage NH is restored to the original shape thereof, and the outer peripheral surface thereof makes contact with the inner surface of the nasal passage NH (including the inner surface of the nasal wing) with a predetermined contact force. In other words, in the present embodiment, the nasal cavity insertion tube 200 is fixed by means of a force of expanding the nasal passage NH from the inside of the nasal passage NH. The fixture 100 is thereby fixed to the inside of the nasal passage NH.

The nasal cavity insertion tube 200 is disposed inside the tubular fixture 100. In short, in the present embodiment, a dual-nested tubular structure is formed by the fixture 100 and the nasal cavity insertion tube 200. A plurality of holding portions 300 are mounted between the fixture 100 and the nasal cavity insertion tube 200. The fixture 100 and the nasal cavity insertion tube 200 are disposed through a predetermined interval by the holding portions 300. An air flow path can be reliably produced by thus disposing the fixture 100 and the nasal cavity insertion tube 200 through the predetermined interval. As a result, hindrance to the respiration of a patient by the fixture 100 and the nasal cavity insertion tube 200 can be suppressed. The holding portions 300 take the form of elastic bodies for outwardly urging the fixture 100, and are composed of springs or rubber parts.

### <Method of Using Nasal Cavity Insertion Device Set>

First, the expansion portion 220 of the nasal cavity insertion tube 200 is gradually inserted to reach the pharynx. Then, the fixture 100 is inserted into the nasal passage NH. At this time, the fixture 100 is inserted into the nasal passage NH while the diameter thereof is reduced. When the insertion of the fixture 100 into the nasal passage NH is finished, the fixture 100 is restored to the original shape thereof and the outer peripheral surface of the fixture 100 makes contact with the inner surface of the nasal passage NH. Accordingly, the nasal cavity insertion tube 200 is fixed to the nasal passage NH by means of the force of expanding the nasal passage NH, i.e., the force applied by the fixture 100.

In removing the fixture 100, it is only required to simply hold the fixture 100 by the hand and pull it down.

### <Features of Nasal Cavity Insertion Device Set and Fixture>

(1) The fixture 100 according to the first embodiment can fix the nasal cavity insertion tube 200 by means of the force of expanding the nasal passage NH from the inside of the nasal passage NH. Therefore, it is possible to prevent the displacement or detachment of the nasal cavity insertion tube 200 during the sleep of a patient of sleep disorder. Especially, in the present embodiment, the outer peripheral surface of the tubular fixture 100 makes contact with the inner peripheral surface of the nasal passage NH by means of surface contact. Therefore, the nasal cavity insertion tube 200 can be firmly fixed.
(2) Further, in the nasal cavity insertion device set, the fixture 100 is disposed inside the nasal passage NH. Therefore, the fixture 100 can be prevented from standing out.
(3) Further, in the nasal cavity insertion device set 1, the attached position of the fixture 100 is the nose. Therefore, a user can attach the fixture 100 thereto by oneself.
(4) Further, the fixture 100 is formed in a tubular shape that allows the air to pass therethrough. Therefore, hindrance to the respiration of a patient by the fixture 100 can be suppressed.
(5) Further, in the nasal cavity insertion device 1, the nasal cavity insertion tube 200 can be accommodated inside the tubular fixture 100. Therefore, a single nasal passage can be commonly used as the nasal passage NH into which the nasal cavity insertion tube 200 is inserted and the nasal passage NH into which the fixture 100 is inserted. A normal nasal cavity, free from occurrence of the obstruction of the pharynx, can be thereby kept open. Therefore, hindrance to the respiration of a patient by the fixture 100 can be suppressed.
(6) Further, in the nasal cavity insertion device set 1, the nasal cavity insertion tube 200 is accommodated inside the tubular fixture 100. Therefore, the nasal cavity insertion tube 200 can be prevented from standing out. Accordingly, a patient can use the nasal cavity insertion tube 200 and the nasal cavity insertion device set 1 without hesitation.

### <Nasal Cavity Insertion Device Set>

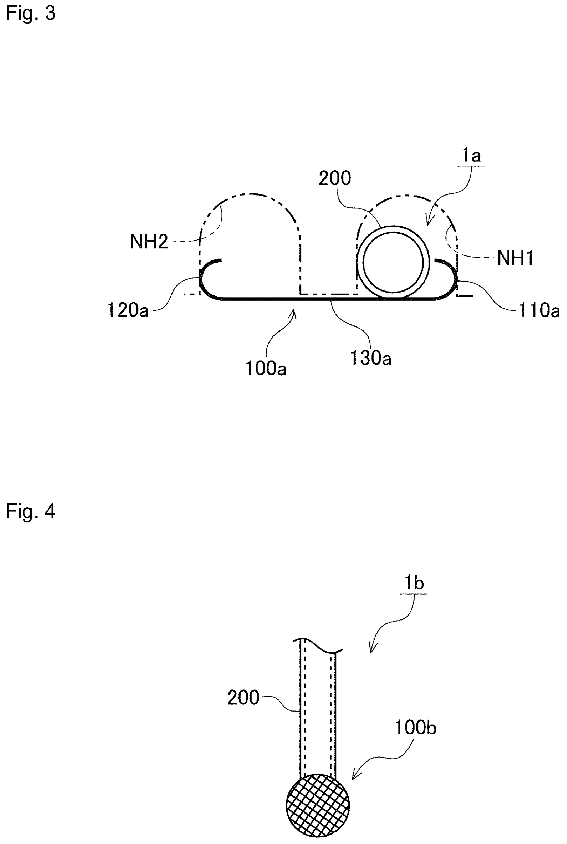

As shown in FIG. 3, a nasal cavity insertion device set 1a according to an example includes the nasal cavity insertion tube 200 and a fixture 100a for fixing the nasal cavity insertion tube 200. The nasal cavity insertion tube 200 is similar to that in the first embodiment. Therefore, the explanation thereof will not be hereinafter made.

### <Fixture>

As shown in FIG. 3, the fixture 100a makes contact with both of the inner surface of a nasal passage NH1 and that of a nasal passage NH2 in order to fix the nasal cavity insertion tube 200 to be connected to the fixture 100a. The fixture 100a is a flat spring and has: a first curved portion 110a making contact with the inner surface of the nasal passage NH1 into which the nasal cavity insertion tube 200 is inserted; a second curved portion 120a making contact with the inner surface of the nasal passage NH2 that is different from the nasal passage NH1; and a coupling portion 130a coupling the first curved portion 110a and the second curved portion 120a. The coupling portion 130a causes the first curved portion 110a to make contact with the inner surface of one nasal wing forming the nasal passage NH1 with a predetermined contact force, while causing the second curved portion 120a to make contact with the inner surface of the other nasal wing forming the nasal passage NH2. In other words, the fixture 100a is set such that it is pressed against and supported between the inner surfaces of the both nasal wings. It should be noted that the fixture 100a, taking the form of the flat spring, may be made of resin, or alternatively, may be made of metal.

The first curved portion 110a is curved for making contact with the inner surface of the nasal passage NH1, while the second curved portion 120a is curved for making contact with the inner surface of the nasal passage NH2. Damage of the inner surfaces of the nasal passages NH1 and NH2 can be prevented by thus curving the first curved portion 110a and the second curved portion 120a. It should be noted that the length of the coupling portion 130a may be adjustable so that the first curved portion 110a and the second curved portion 120a can be located closer to or away from each other. When the length of the coupling portion 130a is designed to be adjustable, the fixture 100a can be fixed regardless of the size of the nose of a patient.

In the present embodiment, the fixture 100a and the nasal cavity insertion tube 200 may be joined by means of welding, or alternatively, by means of adhesion.

In the present embodiment, the first curved portion 110a and the second curved portion 120a respectively press the inner surfaces located away from a nasal bridge NP within the nasal passages NH1 and NH2. In other words, the fixture 100a is pressed against and supported by the both nasal wings. At this time, the fixture 100a as the flat spring exerts an elastic force, and is thereby fixed with a predetermined contact force.

### <Method of Using Nasal Cavity Insertion Device Set>

First, the expansion portion 220 of the nasal cavity insertion tube 200 is gradually inserted to reach the pharynx. Then, the first curved portion 110a of the fixture 100a is inserted into the nasal passage NH1, while the second curved portion 120a is inserted into the nasal passage NH2. Accordingly, the first curved portion 110a presses the inner surface of the nasal passage NH1 (the inner surface of the nasal wing forming the nasal passage NH1) with a predetermined contact force, while the second curved portion 120a presses the inner surface of the nasal passage NH2 (the inner surface of the nasal wing forming the nasal passage NH2) with a predetermined contact force. As a result, the fixture 100a is set such that it is pressed against and supported by the both nasal wings. Accordingly, the nasal cavity insertion tube 200 mounted to the fixture 100a is fixed.

In removing the fixture 100a, it is only required to simply hold the fixture 100a by the hand and pull it down.

### <Features of Nasal Cavity Insertion Device Set and Fixture>

(1) The fixture 100a can fix the nasal cavity insertion tube 200 by means of the force of expanding the nasal passages NH from the inside of the nasal passages NH. Therefore, it is possible to prevent the displacement or detachment of the nasal cavity insertion tube 200 during the sleep of a patient of sleep disorder. Especially, as to the fixture 100a according to the second embodiment, the fixture 100a is fixed by the both nasal passages NH1 and NH2. Therefore, the fixture 100a and the nasal cavity insertion tube 200 mounted thereto can be firmly fixed.
(2) Further, in the nasal cavity insertion device set 1a, the curved portions 110a and 120a of the fixture 100a are respectively disposed inside the nasal passages NH1 and NH2. Therefore, the fixture 100a can be prevented from standing out.
(3) Further, in the nasal cavity insertion device set 1a, the attached position of the fixture 100a is the nose. Therefore, a user can attach the fixture 100a to the nose by oneself.
(4) Further, in the nasal cavity insertion device 1a, the fixture 100a, having the first curved portion 110a, the second curved portion 120a and the coupling portion 130a, can be formed by the single flat spring. In other words, the fixture 100a can be simply structured. Accordingly, in reusing the fixture 100a, the fixture 100a can be easily cleaned and can be used in a sanitary manner.

### <Nasal Cavity Insertion Device Set>

As shown in FIG. 4, a nasal cavity insertion device set 1b according to an example includes the nasal cavity insertion tube 200 and a fixture 100b for fixing the nasal cavity insertion tube 200. The nasal cavity insertion tube 200 is similar to that in the first embodiment. Therefore, the explanation thereof will not be hereinafter made.

### <Fixture>

As shown in FIG. 4, the fixture 100b makes contact with the inner surface of the nasal passage NH (see FIG. 2, this is similarly applied in the following) in order to fix the nasal cavity insertion tube 200 connected to the fixture 100b. The fixture 100b is a reticular member with a roughly spherical contour, and is swelled on the tip end of the nasal cavity insertion tube 200. The outer diameter of the roughly spherical fixture 100b is greater than the inner diameter of the nasal passage. The size of the aforementioned inner diameter of the nasal passage NH depends on patients. Therefore, it is preferable to select the outer diameter of the fixture suitable for a patient on an as-needed basis. Further, in the present embodiment, the fixture 100b can be increased and reduced in the diameter thereof. When inserted into the nasal passage NH, the fixture 100b is inserted into the nasal passage NH while the diameter thereof is reduced. In turn, the fixture 100b inserted into the nasal passage NH is restored to the original shape thereof, and the outer peripheral surface thereof makes contact with the inner surface of the nasal passage NH (including the inner surface of the nasal wing) with a predetermined contact force. In other words, in the present embodiment, the nasal cavity insertion tube 200 is fixed by means of the force of expanding the nasal passage NH from the inside of the nasal passage NH. The fixture 100b is thereby fixed to the inside of the nasal passage NH.

### <Method of Using Nasal Cavity Insertion Device Set>

First, the expansion portion 220 of the nasal cavity insertion tube 200 is gradually inserted to reach the pharynx. Then, the fixture 100b is inserted into the nasal passage NH. At this time, the fixture 100b is inserted into the nasal passage NH while the diameter thereof is reduced. When the insertion of the fixture 100b into the nasal passage NH is finished, the fixture 100b is restored to the original shape thereof and the outer peripheral surface of the fixture 100b makes contact with the inner surface of the nasal passage NH. Accordingly, the nasal cavity insertion tube 200 is fixed to the nasal passage NH by means of the force of expanding the nasal passage NH, i.e., the force applied by the fixture 100b.

In removing the fixture 100b, it is only required to simply hold the fixture 100b by the hand and pull it down.

### <Features of Nasal Cavity Insertion Device Set and Fixture>

(1) The fixture 100b can fix the nasal cavity insertion tube 200 by means of the force of expanding the nasal passage NH from the inside of the nasal passages NH. Therefore, it is possible to prevent the displacement or detachment of the nasal cavity insertion tube 200 during the sleep of a patient of sleep disorder. Especially, in the present embodiment, the reticular portion of the fixture 100b makes contact with the inner peripheral surface of the nasal cavity at multiple points. Therefore, the fixture 100b and the nasal cavity insertion tube 200 mounted to the fixture 100b can be firmly fixed.
(2) Further, in the nasal cavity insertion device set 1b, the fixture 100b is disposed inside the nasal passage NH. Therefore, the fixture 100b can be prevented from standing out.
(3) Further, in the nasal cavity insertion device set 1b, the attached position of the fixture 100b is the nose. Therefore, a user can attach the fixture 100b to the nose by oneself.
(4) Further, the fixture 100b according to this embodiment is the reticular member that allows the air to pass therethrough. Therefore, even when inserted into the nasal passage NH, hindrance to the respiration of a patient by the fixture 100b can be suppressed.

### -Reference Example-

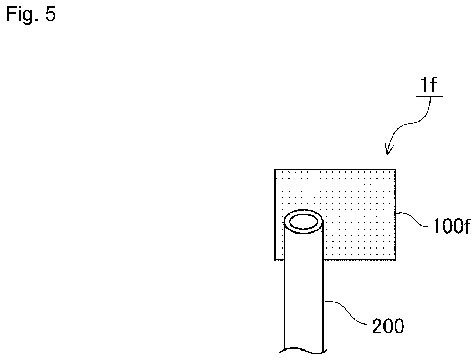

As a reference example of the present invention, the inventors of the present application propose a nasal cavity insertion device set 1f as shown in FIG. 5. The nasal cavity insertion device set 1f includes the nasal cavity insertion tube 200 and an adhesive sheet 100f mounted to the base end of the nasal cavity insertion tube 200. The present nasal cavity insertion tube 200 is similar to the aforementioned nasal cavity insertion tube 200 according to the first embodiment. Therefore, the explanation thereof will not be hereinafter made. Further, in the reference example, the adhesive sheet 100f is adhered to the nose. Accordingly, the nasal cavity insertion tube 200 attached to the adhesive sheet 100f is fixed to the nose.

### <Modifications>

The embodiments and the practical example of the present invention have been explained above based on the drawings. However, it should be understood that the specific configuration should not be limited to the embodiments. The scope of the present invention is represented by claims.

To prevent the fixture from standing out in wearing of the fixture, the color of the fixture may be transparent, the color of skin, pink, or black, although having not been mentioned in the aforementioned embodiments.

Further, silicone resin has been exemplified as the materials of the fixture 100 and the nasal cavity insertion tube 200 in the first embodiment. However, the materials related to the present invention is not limited to this, and other than silicone resin, the following materials can be applied. Specific examples are: ABS resin (acrylonitrile-butadiene-styrene); butadiene-styrene rubber; polyester copolymer; ethylene-propylene rubber (ethylene-propylene-terpolymer rubber); EVA resin (ethylene-vinylacetate copolymer); high-density polyethylene; high-density polypropylene; impact-resistant polystyrene; low-density polyethylene; methylmetacrylate-acrylonitrile-butadiene-styrene copolymer; chloroprene rubber; nitrilebutadiene rubber; polyamide resin; PETG resin; polyacetal resin; polybutyleneterephthalate resin; polycarbonate resin; polyethersulfone resin; polyethylene resin; polyethyleneterephthalate resin; polyimide resin; isobutylene-isoprene copolymer; polypropylene resin; polystyrene resin; polysulfone resin; polytetrafluoroethylene resin; polyurethane resin; polyvinylacetate resin; polyvinylchloride resin; styrene-butadiene resin; styrene-butadiene rubber; acrylic modified silicone resin; and one-component RTV (Room Temperature Vulcanizable) rubber.

### INDUSTRIAL APPLICABILITY

The fixture and the nasal cavity insertion device set according to the present invention are characterized in that the displacement or detachment of the nasal cavity insertion tube can be prevented during the sleep of a patient of sleep disorder.

## Claims

1. A nasal cavity insertion device set (1b), comprising:
a device (200) to be inserted into a nasal cavity, and
a nasal cavity insertion device fixture (100b) configured to fix said device (200) to be inserted by means of a force of expanding a nasal passage from inside the nasal passage, and to make contact with at least an inner surface of a nasal wing, the nasal cavity insertion device fixture (100b) comprising a swell portion whose outer peripheral surface is configured to make contact with an inner surface of the nasal passage,
**characterized in that** the swell portion is a reticular member that allows air to pass therethrough; and
the nasal cavity insertion device fixture (100b) is attached to the tip end of the device (200) to be inserted into the nasal cavity.

2. The nasal cavity insertion device set (1b) recited in claim 1, wherein the swell portion has a roughly spherical contour.

## Patentansprüche

1. Vorrichtungsset (1b) zum Einführen in eine Nasenhöhle, aufweisend:
eine Vorrichtung (200) zum Einführen in eine Nasenhöhle, und
eine Befestigung (100b) für die Einführung in die Nasenhöhle, die eingerichtet ist, die einzuführende Vorrichtung (200) mittels Aufweitung eines Nasengangs von Innen zu befestigen, und mit zumindest einer inneren Oberfläche eines Nasenflügels in Kontakt zu kommen, wobei die Befestigung (100b) für die Einführung in die Nasenhöhle einen Schwellabschnitt aufweist, dessen äußere umlaufende Oberfläche eingerichtet ist, mit einer inneren Oberfläche des Nasengangs in Kontakt zu kommen,
**dadurch gekennzeichnet, dass** der Schwellabschnitt ein netzartiges Element ist, welches den Durchgang von Luft ermöglicht; und
die Befestigung (100b) für die Einführung in die Nasenhöhle an ein vorderes Ende der in die Nasenhöhle einzuführenden Vorrichtung (200) angebracht ist.

2. Vorrichtungsset (1b) zum Einführen in eine Nasenhöhle gemäß Anspruch 1, wobei der Schwellabschnitt einen im Wesentlichen sphärischen Umriss aufweist.

## Revendications

1. Ensemble formant dispositif pour insertion dans une cavité nasale (1b), comprenant :
un dispositif (200) à insérer dans une cavité nasale, et
une attache de dispositif pour insertion dans une cavité nasale (100b), conçue pour fixer ledit dispositif (200) à insérer, au moyen d'une force de dilatation d'une voie nasale depuis l'intérieur de la voie nasale, et pour établir un contact avec au moins une surface intérieure d'une aile du nez,
l'attache de dispositif pour insertion dans une cavité nasale (100b) comprenant une partie de gonflement dont la surface périphérique extérieure est conçue pour établir un contact avec une surface intérieure de la voie nasale,
**caractérisé en ce que** la partie de gonflement est un élément réticulaire qui se laisse traverser par l'air ; et
l'attache de dispositif pour insertion dans une cavité nasale (100b) est attachée à l'extrémité du dispositif (200) à insérer dans la cavité nasale.

2. Ensemble formant dispositif pour insertion dans une cavité nasale (1b) selon la revendication 1, dans lequel la partie de gonflement présente un contour sensiblement sphérique.
